# FASCICULE DE BREVET EUROPEEN

(11) **EP 2 758 042 B1**
(45) Date de publication et mention de la délivrance du brevet: **17.08.2016**
(21) Numéro de dépôt: 12775746.6
(22) Date de dépôt: 20.09.2012
(51) Int. Cl.: A61K 31/045, A61K 31/655, A61K 33/14, A61K 33/42, A61K 49/00, A61P 27/02

(54) **COMPOSITION DE COLORATION OPHTALMIQUE À TOXICITÉ RÉDUITE STABLE ET POUVANT ÊTRE STÉRILISÉE**
STABILE STERILISIERBARE ZUSAMMENSETZUNG VON GERINGER TOXIZITÄT ZUM FÄRBEN DER AUGEN
STABLE, LOW-TOXICITY, STERILIZABLE COMPOSITION FOR OPHTHALMIC DYING

(30) Priorité: 22.09.2011 FR 1102880
(43) Date de publication de la demande: 30.07.2014
(73) Titulaire: Arcadophta, 31100 Toulouse (FR)
(72) Inventeur: REBOUL, Gérard, F-31400 Toulouse (FR)
(74) Mandataire: Cabinet BARRE LAFORGUE & associés
(86) Numéro de dépôt international: PCT/FR2012/052101
(87) Numéro de publication internationale: WO 2013/041810

(56) Documents cités:
- EP-A2- 0 881 324
- WO-A1-99/58159
- WO-A1-2004/035091
- WO-A1-2006/062233
- WO-A1-2006/096913
- WO-A2-2006/128269
- AU-A1- 2007 221 941
- DE-A1- 19 731 464
- GB-A- 2 403 408
- JP-A- 62 151 467
- JP-A- 63 156 719
- JP-A- 2003 096 355
- JP-A- 2005 036 156
- JP-A- 2005 336 286
- US-A1- 2004 092 890
- US-A1- 2008 233 507
- US-A1- 2009 318 321
- DATABASE WPI Week 200543 Thomson Scientific, London, GB; AN 2005-422807 XP002675418, & KR 2005 0014622 A (KWAG N H) 7 février 2005 (2005-02-07)
- C. O. PECKAR; N. KÔRBER: "Canaloplasty for open angle glaucoma: a three years critical evaluation and comparison with viscocanalostomy", SPEKTRUM AUGENHEILKD, vol. 22/4, 2008, pages 240-246,
- O'keeffe M And Nabil M: "The use of mannitol in intraocular surgery. - PubMed - NCBI", Ophthalmic surgery, 1 January 1983 (1983-01-01), pages 55-56, XP055184003, Retrieved from the Internet: URL:http://www.ncbi.nlm.nih.gov/pubmed/640 2747 [retrieved on 2015-04-17]
- KRISTIINA KIVIHARJU ET AL: "Contributions of Biotechnology to the Production of Mannitol", RECENT PATENTS ON BIOTECHNOLOGY, vol. 2, no. 2, 1 June 2008 (2008-06-01), pages 73-78, XP055183986, ISSN: 1872-2083, DOI: 10.2174/187220808784619702
- ROWE RAYMOND C ET AL: "Handbook of pharmaceutical excipients, Mannitol", 1 January 2006 (2006-01-01), HANDBOOK OF PHARMACEUTICAL EXCIPIENTS, PHARMACEUTICAL PRESS [U.A.], GB, PAGE(S) 449 - 453, XP002537758, ISBN: 978-1-58212-058-4
- LI K ET AL: "Trypan blue staining of internal limiting membrane and epiretinal membrane during vitrectomy: Visual results and histopathological findings", BRITISH JOURNAL OF OPHTHALMOLOGY, BMJ PUBLISHING GROUP, GB, vol. 87, no. 2, 1 February 2003 (2003-02-01), pages 216-219, XP002346828, ISSN: 0007-1161, DOI: 10.1136/BJO.87.2.216

## Description

L'invention concerne une composition de coloration comprenant au moins un colorant dans une solution aqueuse.

Dans tout le texte, on désigne par « colorant », toute substance présentant la propriété de communiquer une coloration durable à au moins une matière avec laquelle elle a été mise en contact.

Les colorants utilisés en solution aqueuse (qu'ils soient ou non entièrement solubles dans l'eau) sont des composés organiques, notamment organométalliques, comprenant au moins un groupe chromophore (par exemple azoïque, triphényleméthane, thiazidique, phénylamine...) et qui sont donc toxiques pour les tissus cellulaires et les êtres vivants à partir d'un certain niveau de concentration et/ou de temps d'exposition. Or, de nombreux colorants sont utilisés au contact des tissus cellulaires ophtalmiques, notamment à titre d'agent de coloration sélective à des fins de diagnostic, à titre thérapeutique pour des traitements curatifs ou préventifs, dans le domaine de l'expérimentation clinique ou pharmacologique... Dans ces applications, il est nécessaire d'une part de minimiser la toxicité de la composition susceptible de venir au contact de tissus cellulaires ophtalmiques, d'autre part d'assurer la stérilisation de la composition et sa stabilité dans le temps. Il a été par exemple démontré que le vert d'indocyanine est toxique pour les cellules de la rétine ; ou que le bleu de trypan ou le bleu d'aniline sont toxiques vis-à-vis des cellules endothéliales ou épithéliales.

Pour pallier ce problème, et pour densifier une composition comprenant du bleu brillant G, il a été proposé d'y ajouter du glucose à une concentration de 5 %. Néanmoins, il s'avère qu'une telle composition glucosée n'est pas stable aux températures de stérilisation à l'autoclave, et ne peut pas être stérilisée. En outre, l'inventeur a déterminé qu'avec une concentration de glucose aussi faible, la composition n'est pas non plus stable sur une longue période à température ambiante entraînant la dégradation du colorant.

Également, la publication « Comparative toxicology of trypan blue, brillant blue G, and their combination together with polyethyleneglycol on human pigment épithelial cells » Investigative Ophthamology & Visual Science, June 2011, Vol 52, No 7 enseigne la possibilité de réduire la toxicité d'une composition de bleu de trypan par addition de PEG. Néanmoins, ce polymère est synthétisé à partir d'oxyde d'éthylène très toxique, notamment pour les structures internes de l'oeil. L'obtention d'une composition de PEG pur et parfaitement exempte d'oxyde d'éthylène est en pratique quasi impossible, et en tout cas extrêmement onéreux. En outre, ce polymère est un tensioactif susceptible de favoriser la diffusion de la solution colorée sous la rétine, au risque d'augmenter sa toxicité clinique.

L'invention vise donc à pallier l'ensemble de ces inconvénients en proposant une composition de coloration- notamment ophtalmique et plus particulièrement intraoculaire- comprenant au moins un colorant qui présente une toxicité réduite vis-à-vis des cellules de l'oeil, qui puisse être stérilisée, qui soit stable- notamment à l'état stérilisé- sur une longue période (notamment plusieurs mois) à température ambiante, prête à l'emploi et peu coûteuse.

L'invention vise plus particulièrement à proposer une telle composition dans laquelle le pouvoir colorant de chaque colorant est préservé et non modifié.

L'invention vise aussi à proposer une telle composition qui puisse être utilisée in vivo, notamment qui présente un pH et une osmolarité physiologiques. Plus particulièrement, l'invention vise à proposer une telle composition qui soit compatible avec son utilisation en milieu chirurgical, notamment au cours d'opérations de chirurgie ophtalmique et/ou pour le diagnostic.

Dans tout le texte, on désigne par « milieu ophtalmique » tout élément constitutif d'un oeil humain ou animal ; ce terme couvre donc aussi bien les tissus cellulaires, les membranes, et les fluides ophtalmiques (humeur aqueuse, vitré, larmes). Le terme « polyol » désigne un composé de formule H(HCOH)ₙH, c'est-à-dire un composé hydrocarboné comprenant un groupe hydroxyle lié à chaque atome de carbone. Il est à noter que les oses, qui sont des hydrates de carbone, ne sont pas des polyols au sens de cette définition.

L'invention concerne donc une composition de coloration ophtalmique -notamment intraoculaire - stable apte à être stérilisée à l'autoclave comprenant dans une solution aqueuse :
- au moins un colorant,
- au moins un polyol soluble dans l'eau de formule H(HCOH)ₙH, n étant un entier supérieur à 3 (supérieur ou égal à 4).

L'invention concerne aussi une composition de coloration stable apte à être stérilisée à l'autoclave comprenant dans une solution aqueuse :
- au moins un colorant,
au moins un polyol soluble dans l'eau de formule H(HCOH)ₙH, n étant un entier supérieur à 3 (supérieur ou égal à 4),
pour son utilisation dans une méthode choisie parmi :
- une méthode de traitement chirurgical ophtalmique appliquée au corps humain ou animal dans laquelle ladite composition est appliquée au contact d'un milieu ophtalmique de façon à en réaliser dans des conditions physiologiquement acceptables une coloration sélective au cours de la réalisation d'au moins une opération chirurgicale ophtalmique,
- une méthode de diagnostic appliquée au corps humain ou animal (in vivo).

Contre toute attente, et alors même que les oses tels que le glucose ne permettent pas d'obtenir une composition présentant les qualités requises, l'inventeur a constaté que la simple adjonction dans la composition d'un polyol soluble dans l'eau (de structure proche, mais différente de celle des oses, et de structure différente du PEG) permet de réduire considérablement sa cytotoxicité, sans nuire en aucune façon ni à sa stabilité, ni aux propriétés de chaque colorant, tout en étant compatible avec une stérilisation à l'autoclave et avec l'adjonction d'adjuvants appropriés pour l'utilisation de la composition au contact des milieux ophtalmiques. Ce résultat est d'autant plus surprenant que si les polyols peuvent faire l'objet en eux-mêmes de certaines applications au contact des tissus physiologiques, ils ne sont pas eux-mêmes inoffensifs pour les cellules à partir d'une certaine concentration. Par exemple, le mannitol est au contraire connu pour avoir des effets de rétrécissement des cellules endothéliales au moins à certaines concentrations. Aucune explication ne peut donc être donnée à ce résultat inattendu.

L'invention peut s'appliquer pour tout colorant pouvant être utilisé dans une solution aqueuse et compatible avec son utilisation conjointe avec au moins un polyol. Néanmoins, avantageusement une composition selon l'invention comprend au moins un colorant choisi dans le groupe formé du bleu de trypan, du bleu brillant G, du bleu Evans, du bleu patenté V, du bleu de méthylène, du bleu de bromophénol et du bleu d'aniline. Ces colorants sont en effet particulièrement stables en solution aqueuse, supportent une stérilisation à l'autoclave, et peuvent être utilisés dans de nombreuses applications, notamment par voie topique sur les milieux ophtalmiques- notamment intraoculaires- à des fins de diagnostic ou au cours d'une opération de traitement chirurgical, en particulier dans la chirurgie ophtalmique. Leur combinaison à un polyol selon l'invention en réduit par ailleurs considérablement leur toxicité.

La proportion de chaque colorant dans une composition selon l'invention peut varier en fonction de l'application et de l'effet recherché, notamment de l'intensité de la couleur, dans la limite de la toxicité admissible dans l'application, compte tenu de l'adjonction d'au moins un polyol selon l'invention. Avantageusement, une composition selon l'invention comprend une proportion de chaque colorant acceptable pour une application au contact d'un milieu ophtalmique -notamment au cours d'une opération chirurgicale ophtalmique à titre de composition de coloration sélective de milieux ophtalmiques intraoculaires-. Avantageusement, une composition selon l'invention comprend une proportion de chaque colorant en poids sur volume comprise entre 0,001 % et 2,5 %.

De même, la proportion de chaque polyol dans une composition selon l'invention peut varier, et peut notamment être optimisée en fonction de chaque colorant, de chaque polyol considéré et de l'application envisagée de la composition. Les polyols sont en eux-mêmes physiologiquement acceptables en application topique sur les tissus cellulaires, dans une large gamme de concentrations. Avantageusement, une composition selon l'invention comprend une proportion en polyol(s) acceptable pour une application au contact d'un milieu ophtalmique- notamment au cours d'une opération chirurgicale ophtalmique à titre de composition de coloration sélective de milieux ophtalmiques intraoculaires-. Avantageusement, une composition selon l'invention comprend une proportion de polyol(s) en poids sur volume comprise entre 0,1 % et 5 %.

Par ailleurs, les meilleurs résultats ont été obtenus avec des polyols pour lesquels n est inférieur ou égal à 9. De préférence, chaque polyol est choisi dans le groupe formé de l'érythritol (n=4), du xylitol (n=5), de l'arabitol (n=5), du ribitol (n=5), du sorbitol (n=6), du dulcitol (n=6), du mannitol (n=6), du volemitol (n=7), du maltitol (n=9), de l'isomaltipol (n=9) et du lactitol (n=9). En particulier, avantageusement et selon l'invention au moins un polyol est choisi dans le groupe des hexols (n=6).

Encore plus préférentiellement, une composition selon l'invention comprend le mannitol à titre de polyol.

Par ailleurs, avantageusement, une composition selon l'invention est aussi caractérisée en ce qu'elle comprend en outre une quantité de chlorure de sodium adaptée pour que la composition présente une osmolarité comprise entre 260 mOsmole/KgH₂O et 360 mOsmole/KgH₂O. Avantageusement et selon l'invention la proportion de chlorure de sodium est choisie pour ajuster l'osmolarité de la composition à une valeur comprise entre 280 mOsmole/KgH₂O et 350 mOsmole/KgH₂O, notamment de l'ordre de 310 mOsmole/KgH₂O. Avantageusement et selon l'invention, la proportion en poids sur volume de chlorure de sodium est choisie entre 0,4 % et 0,9 %.

Par ailleurs, avantageusement, une composition selon l'invention est aussi caractérisée en ce qu'elle comprend en outre un tampon adapté pour maintenir un pH entre 7 et 7,5. Avantageusement et selon l'invention ledit tampon est H₂PO₄/HPO₄.

Avantageusement, une composition selon l'invention comprend également de l'eau en quantité qsp.

Avantageusement, une composition selon l'invention est aussi caractérisée en ce qu'elle comprend en outre entre 1 % et 20 % (volume/volume)-notamment de l'ordre de 5 %- d'eau lourde (D₂O₂).

Ainsi, dans une formulation préférentielle, une composition selon l'invention est constituée de 0,001 % à 2,5 % (poids/volume) d'au moins un colorant- de préférence un colorant ou deux colorants -, de 0,1 % à 5 % (poids/volume) d'au moins un polyol soluble dans l'eau - de préférence un hexol tel que le mannitol-, d'une proportion de chlorure de sodium permettant d'ajuster l'osmolarité de la composition à une valeur comprise entre 280 mOsmole/KgH₂O et 350 mOsmole/KgH₂O, d'un tampon adapté pour maintenir le pH de la composition entre 7 et 7,5, d'eau lourde à raison de 1 % à 20 % (volume/volume) et d'eau en quantité qsp.

Une composition selon l'invention peut être stérilisée à l'autoclave. En conséquence, une composition selon l'invention est avantageusement caractérisée en ce qu'elle est stérile.

Une composition selon l'invention est stable dans le temps à température ambiante (résiste en particulier à la chaleur ambiante), la quantité de colorant(s) qu'elle contient et ses propriétés colorantes restant stables pendant plusieurs mois.

Une composition de coloration selon l'invention peut en particulier être exempte de PEG, d'oses - notamment de glucose-, d'anticoagulants - notamment d'héparine-, d'agent anesthésiant- notamment d'agent anesthésiant topique -.

Une composition de coloration selon l'invention peut être utilisée en particulier au contact de milieux ophtalmiques humains ou animaux-notamment intraoculaires-. Une composition selon l'invention peut ainsi être utilisée en application au contact de cellules (endothéliales ou épithéliales), membranes ophtalmiques, fluides ophtalmiques, ou autres tissus de l'oeil humain ou animal in vivo.

Plus particulièrement, une composition de coloration selon l'invention peut être utilisée au contact de milieux ophtalmiques humains ou animaux dans une méthode de traitement d'une pathologie susceptible d'entraîner des troubles de la vision (perte (totale ou partielle) de vision, déformations, voiles...) chez l'homme ou l'animal. Une composition de coloration selon l'invention peut en particulier être utilisée au contact des milieux ophtalmiques intraoculaires humains ou animaux dans une méthode choisie parmi une méthode de traitement chirurgical ophtalmique -notamment choisie parmi une opération de traitement chirurgical de la cataracte, une opération de traitement chirurgical du glaucome, et une opération de traitement chirurgical de la rétine - et une méthode de diagnostic ophtalmique.

L'invention s'étend en particulier à une composition de coloration ophtalmique telle que mentionnée ci-dessus pour son utilisation dans une méthode choisie parmi : une méthode de traitement chirurgical ophtalmique dans laquelle la composition est appliquée au contact d'un milieu ophtalmique ; et une méthode de diagnostic ophtalmique dans laquelle la composition est appliquée au contact d'un milieu ophtalmique humain ou animal (in vivo).

Une composition selon l'invention peut être utilisée pour le diagnostic en permettant de distinguer, et d'identifier par coloration différentielle, certaines portions spécifiques de tissus cellulaires ophtalmiques -notamment des cellules mortes ou des tissus cellulaires ophtalmiques pathogènes ou détériorés- de tissus cellulaires sains. En conséquence, l'invention s'étend à une composition telle que mentionnée ci-dessus pour son utilisation dans une méthode de diagnostic appliquée au corps humain ou animal (in vivo), et en particulier pour son utilisation dans une méthode de diagnostic par coloration différentielle de certaines portions spécifiques de tissus cellulaires ophtalmiques intraoculaires -notamment des tissus pathologiques tels que les membranes épirétiniennes de façon à permettre de les distinguer des tissus cellulaires vivants tels que la rétine-. Une telle composition selon l'invention comprend alors avantageusement au moins un colorant vital, par exemple le bleu de trypan. Ainsi, l'invention s'étend en particulier à une composition telle que mentionnée ci-dessus pour son utilisation dans une méthode de diagnostic appliquée au corps humain ou animal (in vivo) dans laquelle la composition est appliquée au contact de la rétine pour distinguer les membranes pathologiques de la rétine. Une telle méthode de diagnostic intervient en particulier au cours de la réalisation d'une opération chirurgicale ophtalmique de traitement des membranes pathologiques de la rétine.

L'invention s'étend ainsi à l'utilisation d'une composition selon l'invention telle que mentionnée ci-dessus dans une telle méthode de diagnostic appliquée au corps humain ou animal (in vivo). L'invention s'étend également à une méthode de diagnostic ophtalmique (pathologie susceptible d'entraîner des troubles de la vision (perte (totale ou partielle) de vision, déformations, voiles...)) appliquée au corps humain ou animal (in vivo) dans laquelle une composition selon l'invention est appliquée au contact d'un tissu cellulaire ophtalmique -notamment intraoculaire- afin d'identifier par coloration sélective certaines portions spécifiques de ce tissu cellulaire ophtalmique, notamment des cellules mortes lorsque la composition selon l'invention comprend au moins un colorant vital tel que le bleu de trypan ou le bleu Evans, ou d'éventuelles portions pathologiques de ce tissu cellulaire ophtalmique.

Une composition selon l'invention peut aussi avantageusement être utilisée dans une méthode de traitement chirurgical ophtalmique en permettant de visualiser par coloration sélective certains fluides physiologiques ophtalmiques ou tissus ophtalmiques (tissus composés de cellules épithéliales et/ou endothéliales ou de membranes basales).

L'invention s'étend donc à l'utilisation d'une composition selon l'invention telle que mentionnée ci-dessus dans une méthode de traitement chirurgical ophtalmique d'une pathologie susceptible d'entraîner des troubles de la vision (perte (totale ou partielle) de vision, déformations, voiles...), appliquée au corps humain ou animal, méthode dans laquelle ladite composition est appliquée au contact d'un milieu ophtalmique -notamment intraoculaire-, de façon à en réaliser dans des conditions physiologiquement acceptables une coloration sélective permettant de le visualiser au cours de la réalisation d'une opération de traitement chirurgical ophtalmique. Elle s'étend donc en particulier à une composition telle que mentionnée ci-dessus pour son utilisation dans une méthode de traitement chirurgical ophtalmique d'une pathologie susceptible d'entraîner des troubles de la vision (perte (totale ou partielle) de vision, déformations, voiles...) appliquée au corps humain ou animal, méthode dans laquelle ladite composition est appliquée au contact d'un milieu ophtalmique -notamment intraoculaire- de façon à en réaliser dans des conditions physiologiquement acceptables une coloration sélective au cours de la réalisation d'au moins une opération chirurgicale ophtalmique. L'invention s'étend également à une méthode de traitement chirurgical ophtalmique d'une pathologie susceptible d'entraîner des troubles de la vision (perte (totale ou partielle) de vision, déformations, voiles...) appliquée au corps humain ou animal, méthode dans laquelle une composition selon l'invention telle que mentionnée ci-dessus est appliquée au contact d'un milieu ophtalmique de façon à en réaliser dans des conditions physiologiquement acceptables une coloration sélective permettant de le visualiser au cours de la réalisation d'une opération chirurgicale ophtalmique.

L'invention s'étend aussi à l'utilisation d'une composition selon l'invention telle que mentionnée ci-dessus dans une méthode de traitement chirurgical ophtalmique appliquée au corps humain ou animal dans laquelle la composition est appliquée au contact de la surface externe de la capsule antérieure de l'oeil de façon à réaliser dans des conditions physiologiquement acceptables une coloration sélective de la capsule antérieure par rapport au tissu lenticulaire sous-jacent facilitant la réalisation de l'ouverture contrôlée de la capsule antérieure au cours d'une opération chirurgicale d'extraction de la cataracte. Elle s'étend donc en particulier à une composition telle que mentionnée ci-dessus pour son utilisation dans une méthode de traitement chirurgical ophtalmique appliquée au corps humain ou animal dans laquelle la composition est appliquée au contact de la surface externe de la capsule antérieure de l'oeil de façon à réaliser dans des conditions physiologiquement acceptables une coloration sélective de la capsule antérieure par rapport au tissu lenticulaire sous-jacent facilitant la réalisation de l'ouverture contrôlée de la capsule antérieure au cours d'une opération chirurgicale d'extraction de la cataracte. L'invention s'étend également à une méthode de traitement chirurgical ophtalmique appliquée au corps humain ou animal dans laquelle la composition est appliquée au contact de la surface externe de la capsule antérieure de l'oeil de façon à réaliser dans des conditions physiologiquement acceptables une coloration sélective de la capsule antérieure par rapport au tissu lenticulaire sous-jacent facilitant la réalisation de l'ouverture contrôlée de la capsule antérieure au cours d'une opération chirurgicale d'extraction de la cataracte.

L'invention s'étend aussi à l'utilisation d'une composition selon l'invention telle que mentionnée ci-dessus dans une méthode de traitement chirurgical ophtalmique appliquée au corps humain ou animal dans laquelle la composition est appliquée au contact de la rétine pour distinguer les membranes pathologiques de la rétine au cours de la réalisation d'une opération chirurgicale ophtalmique de traitement des membranes pathologiques de la rétine. Elle s'étend donc en particulier à une composition telle que mentionnée ci-dessus pour son utilisation dans une méthode de traitement chirurgical ophtalmique appliquée au corps humain ou animal dans laquelle la composition est appliquée au contact de la rétine pour distinguer les membranes pathologiques de la rétine au cours de la réalisation d'une opération chirurgicale ophtalmique de traitement des membranes pathologiques de la rétine. L'invention s'étend également à une méthode de traitement chirurgical ophtalmique appliquée au corps humain ou animal dans laquelle la composition est appliquée au contact de la rétine pour distinguer les membranes pathologiques de la rétine au cours de la réalisation d'une opération chirurgicale ophtalmique de traitement des membranes pathologiques de la rétine.

L'invention s'étend aussi à l'utilisation d'une composition selon l'invention telle que mentionnée ci-dessus dans une méthode de traitement chirurgical ophtalmique appliquée au corps humain ou animal dans laquelle ladite composition est appliquée au contact d'un fluide ophtalmique de façon à en réaliser dans des conditions physiologiquement acceptables une coloration sélective permettant de visualiser la diffusion du fluide ophtalmique au cours de la réalisation d'une opération chirurgicale ophtalmique. Elle s'étend donc en particulier à une composition telle que mentionnée ci-dessus pour son utilisation dans une méthode de traitement chirurgical ophtalmique appliquée au corps humain ou animal dans laquelle ladite composition est appliquée au contact d'un fluide ophtalmique de façon à en réaliser dans des conditions physiologiquement acceptables une coloration sélective permettant de visualiser la diffusion du fluide ophtalmique au cours de la réalisation d'une opération chirurgicale ophtalmique. L'invention s'étend également à une méthode de traitement chirurgical ophtalmique appliquée au corps humain ou animal dans laquelle ladite composition est appliquée au contact d'un fluide ophtalmique de façon à en réaliser dans des conditions physiologiquement acceptables une coloration sélective permettant de visualiser la diffusion du fluide ophtalmique au cours de la réalisation d'une opération chirurgicale ophtalmique. Dans cette application, ladite opération chirurgicale ophtalmique est avantageusement choisie dans le groupe constitué de la chirurgie du glaucome par sclérectomie, de la chirurgie du glaucome par trabéculectomie, de la chirurgie du glaucome par trabéculoplastie, de la chirurgie du glaucome par canaloplastie, et de la chirurgie du glaucome par iridectomie. Ledit fluide ophtalmique est alors l'humeur aqueuse.

L'invention s'étend à l'utilisation d'au moins un colorant et d'au moins un polyol -notamment comme mentionné ci-dessus- pour la fabrication d'une composition de coloration ophtalmique -notamment destinée à être appliquée au contact de la surface externe de la capsule antérieure de l'oeil de façon à réaliser dans des conditions physiologiquement acceptables une coloration sélective de la capsule antérieure par rapport au tissu lenticulaire sous-jacent facilitant la réalisation de l'ouverture contrôlée de la capsule antérieure au cours d'une opération chirurgicale d'extraction de la cataracte-.

L'invention s'étend à l'utilisation d'au moins un colorant et d'au moins un polyol -notamment comme mentionné ci-dessus- pour la fabrication d'une composition pour le diagnostic des membranes pathologiques de la rétine par coloration sélective lors d'une opération de traitement des membranes pathologiques de la rétine.

L'invention concerne également une composition selon l'invention telle que mentionnée ci-dessus ; son utilisation pour la coloration sélective des milieux ophtalmiques, in vivo, ex vivo, ou in vitro, notamment dans une méthode de diagnostic d'une pathologie susceptible d'entraîner des troubles de la vision (perte (totale ou partielle) de vision, déformations, voiles...) appliquée au corps humain ou animal ou dans une méthode de traitement chirurgical d'une pathologie susceptible d'entraîner des troubles de la vision (perte (totale ou partielle) de vision, déformations, voiles...) appliquée au corps humain ou animal ; une telle méthode ; et l'utilisation d'au moins un colorant et d'au moins un polyol tel que mentionné ci-dessus pour la fabrication d'une composition de coloration ophtalmique, caractérisées en combinaison par tout ou partie des caractéristiques mentionnées ci-dessus ou ci-après.

D'autres buts, caractéristiques et avantages de l'invention apparaîtront à la lecture des exemples qui suivent donnés à titre non limitatif.

### Exemple 1

Trois solutions référencées respectivement solution 1, solution 2, solution 3 sont préparées.

La solution 3 (non conforme à l'invention) est constituée de bleu de trypan dissout à 0,15% (poids sur volume) dans une solution saline aqueuse tamponnée comprenant 0,8% (poids sur volume) NaCl et le tampon H₂PO₄/HPO₄, sous forme NaH₂PO₄, 2H₂O à 0,03% ; Na₂HPO₄, 2H₂O à 0,18%.

La solution 1 selon l'invention correspond à la solution 3 à laquelle est ajoutée 1,8% (poids sur volume) de mannitol et la quantité de NaCl ramenée à 0,5%.

La solution 2 (non conforme à l'invention) diffère de la solution 1 par le fait que le mannitol est remplacé par du glucose.

Les solutions 1, 2 et 3 ont un pH physiologique, c'est-à-dire compris entre 7 et 7,5. Elles ont également une osmolarité physiologique, c'est-à-dire comprise entre 280 et 350 mOsmole/KgH20.

On évalue les effets de ces solutions vis-à-vis de cellules endothéliales cornéennes humaines.

On mesure le taux de cytolyse par incorporation d'une sonde fluorescente se fixant sur l'ADN dans les cellules dont la membrane plasmatique est endommagée. La cytolyse est évaluée par le canal de fluorescence approprié d'un cytomètre de flux.

En outre, on mesure le taux de cellules conservant une différence de potentiel membranaire (ΔΨ) mitochondrial. Pour ce faire, on utilise des sondes fluorescentes cationiques non toxiques qui sont accumulées par les mitochondries polarisées selon la valeur résiduelle de leur potentiel membranaire mitochondrial. Elles sont extraites des cellules lorsque la membrane mitochondriale disparaît complètement. Les cellules sont analysées dans des plaques à 96 puits par un cytomètre de flux.

On utilise une lignée de cellules rétiniennes épithéliales ARPE-19 fournie par ATCC.

Ces cellules sont cultivées dans un milieu de culture (mélange en proportion égale de milieu de culture MEM et de milieu de culture de Ham F 12 auquel on ajoute 10 % de sérum foetal bovin et 2 mM de L.-glutamine. Ces cellules sont injectées à raison de 10 000 cellules par puits dans les plaques à 96 puits.

Les cellules sont ensuite placées pendant 2 min au contact de 50 µL des différentes solutions, de façon à mimer les conditions d'une opération chirurgicale. Les solutions sont ensuite lavées et remplacées par du milieu de culture cellulaire frais. Des mesures de cytolyse et du taux de cellules conservant une différence de potentiel membranaire mitochondrial sont réalisées 1,5 heures et 24 heures après ce traitement.

Les mêmes mesures sont effectuées à titre de comparaison sur un milieu de culture cellulaire de référence (milieu de culture MEM) et sur une solution saline ophtalmique d'irrigation intraoculaire (HBSS).

Trois essais sont réalisés pour chaque test. Les résultats sont donnés dans le tableau suivant.

| % de cellules cytolysées en 1,5h | essai 1 | essai 2 | essai 3 | Moyenne |
|---|---|---|---|---|
| Milieu MEM | 19,3 | 20,3 | 6,3 | 15,30 |
| HBSS | 40,4 | 26,2 | 6,2 | 24,27 |
| Solution 3 | 33,3 | 33,6 | 10,6 | 25,83 |
| Solution 2 | 33,8 | 14 | 7,8 | 18,53 |
| Solution 1 | 22,4 | 8,3 | 10,5 | 13,73 |

| % de cellules cytolysées en 24h | | | | |
|---|---|---|---|---|
| Milieu MEM | 18,3 | 16,4 | 10,2 | 14,97 |
| HBSS | *25*,*4* | 13,1 | 6,6 | 15,03 |
| Solution 3 | 13,7 | 7,2 | 9,6 | 10,17 |
| Solution 2 | 19,6 | 11,2 | 7,7 | 12,83 |
| Solution 1 | *20*,*2* | 7,9 | 9,8 | 12,63 |

| % de cellules ayant des mitochondries polarisées après 1,5h | | | | |
|---|---|---|---|---|
| Milieu MEM | 88 | 93,8 | 97,7 | 93,17 |
| HBSS | 62,9 | 70,3 | *95,9* | 76,37 |
| Solution 3 | 65,4 | 54,2 | *97,3* | 72,30 |
| Solution 2 | *47* | 95,1 | 94,7 | 78,93 |
| Solution 1 | 83,7 | 96,2 | 89,9 | 89,93 |

| % de cellules ayant des mitochondries polarisées après 24h | | | | |
|---|---|---|---|---|
| Milieu MEM | 90,6 | 92,6 | 95,8 | 93,00 |
| HBSS | 95,6 | 95,1 | 98 | 96,23 |
| Solution 3 | 96,6 | 96,2 | 95,9 | 96,23 |
| Solution 2 | 96,9 | 96,6 | 97 | 96,83 |
| Solution 1 | 95,6 | 96,1 | 95,6 | 95,77 |

La solution 1 présente une cytotoxicité équivalente à celle du milieu de culture cellulaire de référence (13,7% et 15,3%).

De plus le taux de cellules ayant un ΔΨ mitochondrial est très proche de celui du milieu de culture cellulaire de référence (89,9% et 93,2%).

On constate donc une absence surprenante de cytotoxicité de la solution 1 selon l'invention.

La solution 3, solution saline avec 0,15% de bleu de trypan sans mannitol, présente une cytotoxicité accrue par rapport au milieu de culture cellulaire de référence (25,8% et 15,3%) ainsi qu'un taux de cellule ayant un ΔΨ mitochondrial, sensiblement diminué par rapport à celui correspondant au milieu de culture cellulaire (72,3% et 93,2%).

La solution 2 présente une cytotoxicité intermédiaire entre celle obtenue par le milieu de culture cellulaire de référence et celle de la solution 3 (respectivement 18,5% ; 15,3% et 25,8%).

De même le taux de cellules ayant un ΔΨ mitochondrial est intermédiaire entre celui du milieu de culture cellulaire de référence et celui de la solution 3 (respectivement 78,9% ; 93,2% et 72,3%).

### Exemple 2 : stabilité après stérilisation

La solution 1 a été stérilisée à l'autoclave (121°C 20min) et est ainsi prête à l'emploi. Cette stérilisation ne produit aucune impureté détectable, et ne modifie pas sensiblement les spectres d'absorption aux ultraviolets ou dans le visible.

La solution 1 selon l'invention est stable à température ambiante (+4°C /+30°C), la quantité de colorant qu'elle contient mesurée par chromatographie en phase gazeuse restant constante pendant plusieurs mois.

La solution 3 contenant uniquement du bleu de trypan est également stable de la même manière, y compris après stérilisation.

Au contraire, après stérilisation, la solution 2 contenant du glucose est modifiée : son pH diminue de 7,36 à 6,2 et l'aire de son pic d'absorption à 595 nm diminue d'environ 20 %.

### Exemple 3 :

On a préparé des solutions identiques à la solution 1 mais en remplaçant le bleu de trypan par un colorant choisi parmi le bleu Evans, le bleu brilliant G, le bleu de méthylène et le bleu d'aniline.

Toutes ces solutions ont pu être stérilisées à l'autoclave (121°C 20min). Là encore, il a été constaté que chacune de ces solutions est stable à température ambiante (+4°C /+30°C), la quantité de colorant qu'elle contient mesurée par chromatographie en phase gazeuse restant constante pendant plusieurs mois.

Par ailleurs, le mannitol peut être remplacé par tout autre polyol biocompatible et stable, notamment choisi dans le groupe formé de l'érythritol (n=4), du xylitol (n=5), de l'arabitol (n=5), du ribitol (n=5), du sorbitol (n=6), du dulcitol (n=6), du volemitol (n=7), du maltitol (n=9), de l'isomaltipol (n=9) et du lactitol (n=9).

### Exemple 4 :

Dans cet essai, on évalue également l'influence de la présence d'eau lourde dans les solutions sur leur cytotoxicité vis-à-vis de cellules rétiniennes épithéliales ARPE-19 fournies par ATCC, dans les mêmes conditions générales que dans l'exemple 1.

Les cellules sont exposées aux solutions 30 minutes dans une première série d'essais, et cinq minutes dans une deuxième série d'essais. Des mesures de cytolyse sont réalisées 1,5 h et 48 h après ce traitement.

La solution 4 selon l'invention est constituée de bleu de trypan à 0,15% (poids sur volume), de 2,5g/L de mannitol dans une solution saline aqueuse tamponnée comprenant 0,45g/L NaCl et le tampon H₂PO₄/HPO₄, sous forme NaH₂PO₄, 2H₂O à 0,03% ; Na₂HPO₄, 2H₂O à 0,18%.

La solution 5 selon l'invention est constituée de bleu de trypan à 0,15% (poids sur volume), de 2,5g/L de mannitol dans une solution saline aqueuse tamponnée comprenant 5 % (volume sur volume) d'eau lourde, 0,45g/L NaCl et le tampon H₂PO₄/HPO₄, sous forme NaH₂PO₄, 2H₂O à 0,03% ; Na₂HPO₄, 2H₂O à 0,18%.

Les mêmes mesures sont effectuées à titre de comparaison sur composition de Monoblue® 0,15% commercialisée par la société Arcadophta, Toulouse, France, sur une solution d'eau oxygénée 100mM et sur une solution saline d'irrigation intraoculaire (BSS).

Les résultats obtenus sont donnés dans les tableaux suivants :

**culture 1 h30 après exposition**

| | Exposition 30 min | | | |
|---|---|---|---|---|
| | Nombre de cellules | | % Cellules cytolysées | |
| | Moyenne | Ecart type | Moyenne | Ecart type |
| BSS | 663,33 | 143,62 | 1,27% | 0,61% |
| H2O2 100mM | 29,33 | 5,51 | 73,63% | 8,37% |
| Solution 4 | 322,00 | 35,79 | 2,20% | 2,48% |
| Solution 5 | 295,00 | 67,56 | 1,23% | 1,02% |
| Solution 3 | 384,33 | 72,06 | 1,83% | 1,60% |

**culture 48 h après exposition**

| | Exposition 5 min | | | |
|---|---|---|---|---|
| | Nombre de cellules | | % Cellules cytolysées | |
| | Moyenne | Ecart type | Moyenne | Ecart type |
| BSS | 4786,67 | 919,57 | 5,4% | 1,2% |
| H2O2 100mM | 129,00 | 44,44 | 88,3% | 2,9% |
| Monoblue® 0.15 | 3689,33 | 1584,45 | 3,5% | 1,2% |
| Solution 4 | 3725,33 | 491,24 | 3,0% | 1,7% |
| Solution 5 | 2335,67 | 889,52 | 2,5% | 0,8% |
| Solution 3 | 2119,00 | 284,99 | 4,1% | 1,2% |

Les solutions ont été stérilisées à l'autoclave et ont pu être conservées durant 59 jours à 55° C, et à 40° C pendant six mois, correspondant selon la loi d'Arrhénius à un vieillissement accéléré de deux ans à 20° C. L'analyse des solutions a été effectuée par mesure HPLC. L'aire du pic d'absorption de la solution 4 n'est pas modifiée au cours du temps, celle du pic d'absorption de la solution 5 est, après vieillissement, encore de 98,20 % à celle de la solution initiale. Le pH des deux solutions reste sensiblement constant au cours du temps. Les solutions selon l'invention conservent donc bien la stabilité du colorant dans le temps. Les solutions 4 et 5 selon l'invention induisent moins de cytolyse que la solution 3 de comparaison non conforme à l'invention, et que la composition commerciale de bleu de trypan. En outre, de façon surprenante, la présence d'eau lourde diminue sensiblement la cytolyse. On constate également qu'une composition selon l'invention résiste particulièrement bien à la chaleur et peut donc être conservée sur une longue durée sans précautions particulières vis-à-vis de la température.

Une composition selon l'invention peut être utilisée en particulier avantageusement dans une méthode de traitement chirurgical du corps humain ou animal choisie parmi :
- une méthode de traitement chirurgical ophtalmique d'extraction de la cataracte dans laquelle la composition est appliquée au contact de la surface externe de la capsule antérieure de l'oeil (cf. par exemple « Trypan blue dye in extra-capsular cataract surgery : initial observations », J. M. Waziri-Erameh et al, Ann Biomed Sci., Vol.5 Nos 1& 2, June & December 2006 : 1-4),
- une méthode de traitement chirurgical ophtalmique de traitement des membranes pathologiques de la rétine dans laquelle la composition est appliquée au contact de la rétine (cf. par exemple « Trypan blue staining of internal limiting membrane and epiretinal membrane during vitrectomy: visual results and histopathological findings », K Li, D Wong, P Hiscott, P Stanga, C Groenewald, J McGalliard, Br J Ophthalmol 2003;87:216-219),
- une méthode de traitement chirurgical ophtalmique choisie dans le groupe constitué de la chirurgie du glaucome par sclérectomie (cf. par exemple vidéo V3018 présentée lors du congrès de l'APAO (« Asian & Pacific Association of Ophthalmology ») Singapour 2006, Asian Journal of Ophthalmology Volume 8 N°3 supplement 1 page 385) ; de la chirurgie du glaucome par trabéculectomie (cf. par exemple « Use of Trypan Blue to confirm the patency of filtering surgery », Shishir Agrawal et al, Journal of Cataract & Refractive Surgery Vol. 31, Issue 1, January 2005 235-237) ; de la chirurgie du glaucome par trabéculoplastie, de la chirurgie du glaucome par canaloplastie (cf. par exemple « Canaloplasty for open angle glaucoma: a three years critical évaluation and comparison with viscocanalostomy », C. O. Peckar and N. Körber, Spektrum Augenheilkd (2008) 22/4: 240-246) ; et de la chirurgie du glaucome par iridectomie. Dans ces opérations de traitement chirurgical du glaucome, la composition de coloration est appliquée au contact de l'humeur aqueuse.

## Revendications

1. - Composition de coloration stable apte à être stérilisée à l'autoclave comprenant dans une solution aqueuse :
- au moins un colorant,
- au moins un polyol soluble dans l'eau de formule H(HCOH)ₙH, n étant un entier supérieur à 3,
pour son utilisation dans une méthode choisie parmi :
- une méthode de traitement chirurgical ophtalmique appliquée au corps humain ou animal dans laquelle ladite composition est appliquée au contact d'un milieu ophtalmique de façon à en réaliser dans des conditions physiologiquement acceptables une coloration sélective au cours de la réalisation d'au moins une opération chirurgicale ophtalmique,
- une méthode de diagnostic appliquée au corps humain ou animal (in vivo).

2. - Composition selon la revendication 1, **caractérisée en ce qu'**elle comprend au moins un colorant choisi dans le groupe formé du bleu de trypan, du bleu brillant G, du bleu Evans, du bleu patenté V, du bleu de méthylène, du bleu de bromophénol et du bleu d'aniline.

3. - Composition selon l'une des revendications 1 ou 2, **caractérisée en ce qu'**elle comprend une proportion de chaque colorant acceptable pour une application au contact d'un milieu ophtalmique.

4. - Composition selon l'une des revendications 1 à 3, **caractérisée en ce qu'**elle comprend une proportion de chaque colorant en poids sur volume comprise entre 0,001 % et 2,5 %.

5. - Composition selon l'une des revendications 1 à 4, **caractérisée en ce qu'**elle comprend une proportion en polyol(s) acceptable pour une application au contact d'un milieu ophtalmique.

6. - Composition selon l'une des revendications 1 à 5, **caractérisée en ce qu'**elle comprend une proportion de polyol(s) en poids sur volume comprise entre 0,1 % et 5 %.

7. - Composition selon l'une des revendications 1 à 6, **caractérisée en ce que** n est inférieur ou égal à 9.

8. - Composition selon l'une des revendications 1 à 7, **caractérisée en ce que** chaque polyol est choisi dans le groupe formé de l'érythritol (n=4), du xylitol (n=5), de l'arabitol (n=5), du ribitol (n=5), du sorbitol (n=6), du dulcitol (n=6), du mannitol (n=6), du volemitol (n=7), du maltitol (n=9), de l'isomaltipol (n=9) et du lactitol (n=9).

9. - Composition selon l'une des revendications 1 à 8, **caractérisée en ce qu'**elle comprend le mannitol à titre de polyol.

10. - Composition selon l'une des revendications 1 à 9, **caractérisée en ce qu'**elle comprend en outre une quantité de chlorure de sodium adaptée pour que la composition présente une osmolarité comprise entre 260 et 360 mOsmole/KgH₂O.

11. - Composition selon la revendication 10, **caractérisée en ce qu'**elle comprend une proportion en poids sur volume de chlorure de sodium comprise entre 0,4 % et 0,9 %.

12. - Composition selon l'une des revendications 1 à 11, **caractérisée en ce qu'**elle comprend en outre un tampon adapté pour maintenir un pH entre 7 et 7,5.

13. - Composition selon la revendication 12, **caractérisée en ce que** le tampon est H₂PO₄/HPO₄.

14. - Composition selon l'une des revendications 1 à 13, **caractérisée en ce qu'**elle est constituée de 0,001 % à 2,5 % (poids/volume) d'au moins un colorant, de 0,1 % à 5 % (poids/volume) d'au moins un polyol soluble dans l'eau, d'une proportion de chlorure de sodium permettant d'ajuster l'osmolarité de la composition à une valeur comprise entre 280 mOsmole/KgH₂O et 350 mOsmole/KgH₂O, d'un tampon adapté pour maintenir le pH de la composition entre 7 et 7,5, d'eau lourde à raison de 1 % à 20 % (volume/volume) et d'eau en quantité qsp.

15. - Composition selon l'une des revendications 1 à 14, **caractérisée en ce qu'**elle est stérile.

16. - Composition selon l'une des revendications 1 à 15 pour son utilisation dans une méthode de traitement chirurgical du corps humain ou animal choisie parmi :
- une méthode de traitement chirurgical ophtalmique d'extraction de la cataracte dans laquelle la composition est appliquée au contact de la surface externe de la capsule antérieure de l'oeil,
- une méthode de traitement chirurgical ophtalmique de traitement des membranes pathologiques de la rétine dans laquelle la composition est appliquée au contact de la rétine,
- une méthode de traitement chirurgical ophtalmique du glaucome.

17. - Composition selon l'une des revendications 1 à 15 pour son utilisation dans une méthode de diagnostic ophtalmique appliquée au corps humain ou animal par coloration différentielle de certaines portions spécifiques de milieux ophtalmiques - notamment intraoculaires-.

18. - Composition selon l'une des revendications 1 à 15 pour son utilisation dans une méthode de diagnostic, par coloration différentielle, des membranes pathologiques de la rétine.

## Patentansprüche

1. Stabile Zusammensetzung zum Färben, die im Autoklav sterilisiert werden kann, umfassend in einer wässrigen Lösung:
- mindestens einen Farbstoff,
- mindestens ein wasserlösliches Polyol mit der Formel H(HCOH)ₙH, wobei n eine ganze Zahl größer als 3 ist,
für ihre Verwendung in einem Verfahren, ausgewählt aus:
- einem Verfahren zur chirurgischen ophthalmischen Behandlung, angewendet auf den menschlichen oder tierischen Körper, wobei die Zusammensetzung im Kontakt mit einer ophthalmischen Umgebung angewendet wird, um unter physiologisch annehmbaren Bedingungen eine selektive Färbung im Laufe der Durchführung mindestens eines chirurgischen ophthalmischen Eingriffs durchzuführen,
- ein Diagnoseverfahren, angewendet auf den menschlichen oder tierischen Körper (*in vivo*).

2. Zusammensetzung nach Anspruch 1, **dadurch gekennzeichnet, dass** sie mindestens einen Farbstoff umfasst, ausgewählt aus der Gruppe, gebildet aus Trypanblau, Brillant Blau G, Evansblau, Patentblau V, Methylenblau, Bromphenolblau und Anilinblau.

3. Zusammensetzung nach einem der Ansprüche 1 oder 2, **dadurch gekennzeichnet, dass** sie eine Proportion jedes annehmbaren Farbstoffs für eine Anwendung im Kontakt mit einer ophthalmischen Umgebung umfasst.

4. Zusammensetzung nach einem der Ansprüche 1 bis 3, **dadurch gekennzeichnet, dass** sie eine Proportion jedes Farbstoffs in Gewicht pro Volumen umfasst, die zwischen 0,001 % und 2,5 % liegt.

5. Zusammensetzung nach einem der Ansprüche 1 bis 4, **dadurch gekennzeichnet, dass** sie eine Proportion aus annehmbarem/annehmbaren Polyol(en) für eine Anwendung im Kontakt mit der ophthalmischen Umgebung umfasst.

6. Zusammensetzung nach einem der Ansprüche 1 bis 5, **dadurch gekennzeichnet, dass** sie eine Proportion von Polyol(en) in Gewicht pro Volumen umfasst, die zwischen 0,1 % und 5 % liegt.

7. Zusammensetzung nach einem der Ansprüche 1 bis 6, **dadurch gekennzeichnet, dass** n kleiner oder gleich 9 ist.

8. Zusammensetzung nach einem der Ansprüche 1 bis 7, **dadurch gekennzeichnet, dass** jedes Polyol ausgewählt ist aus der Gruppe, gebildet aus Erythritol (n = 4), Xylitol (n = 5), Arabitol (n = 5), Ribitol (n = 5), Sorbitol (n = 6), Dulcitol (n = 6), Mannitol (n = 6), Volemitol (n = 7), Maltitol (n = 9), Isomaltipol (n = 9) und Lactitol (n = 9).

9. Zusammensetzung nach einem der Ansprüche 1 bis 8, **dadurch gekennzeichnet, dass** sie Mannitol als Polyol umfasst.

10. Zusammensetzung nach einem der Ansprüche 1 bis 9, **dadurch gekennzeichnet, dass** sie außerdem eine Menge an Natriumchlorid umfasst, die ausgelegt ist, damit die Zusammensetzung eine Osmolarität aufweist, die zwischen 260 und 360 mosmol/KgH₂O liegt,

11. Zusammensetzung nach Anspruch 10, **dadurch gekennzeichnet, dass** sie eine Proportion von Natriumchlorid in Gewicht pro Volumen umfasst, das zwischen 0,4 % und 0,9 % liegt.

12. Zusammensetzung nach einem der Ansprüche 1 bis 11, **dadurch gekennzeichnet, dass** sie außerdem einen Puffer umfasst, der ausgelegt ist, um einen pH-Wert zwischen 7 und 7,5 beizubehalten.

13. Zusammensetzung nach Anspruch 12, **dadurch gekennzeichnet, dass** der Puffer H₂PO₄/HPO₄ ist.

14. Zusammensetzung nach einem der Ansprüche 1 bis 13, **dadurch gekennzeichnet, dass** sie aus 0,001 % bis 2,5 % (Gewicht/Volumen) mindestens eines Farbstoffs, aus 0,1 % bis 5 % (Gewicht/Volumen) mindestens eines wasserlöslichen Polyols, einer Proportion aus Natriumchlorid, die ermöglicht, die Osmolarität der Zusammensetzung auf einen Wert einzustellen, der zwischen 280 mosmol/KgH₂O und 350 mosmol/KgH₂O liegt, einen Puffer, der ausgelegt ist, um den pH-Wert der Zusammensetzung zwischen 7 und 7,5 beizubehalten, schweres Wasser im Umfang von 1 % bis 20 % (Volumen/Volumen) und Wasser *quantum satis* enthält.

15. Zusammensetzung nach einem der Ansprüche 1 bis 14, **dadurch gekennzeichnet, dass** sie steril ist.

16. Zusammensetzung nach einem der Ansprüche 1 bis 15 für ihre Verwendung in einem Verfahren zur chirurgischen Behandlung des menschlichen oder tierischen Körpers, ausgewählt aus:
- einem Verfahren zur chirurgischen ophthalmischen Behandlung zur Extraktion der Katarakt, wobei die Zusammensetzung im Kontakt mit der äußeren Fläche der vorderen Kapsel des Auges angewendet wird.
- einem Verfahren zur chirurgischen ophthalmischen Behandlung zur Behandlung der pathologischen Membrane der Retina, wobei die Zusammensetzung im Kontakt mit der Retina angewendet wird,
- einem Verfahren zur chirurgischen ophthalmischen Behandlung des Glaukoms.

17. Zusammensetzung nach einem der Ansprüche 1 bis 15 für ihre Verwendung bei einem Verfahren zur ophthalmischen Diagnose, angewendet auf den menschlichen oder tierischen Körper durch differenzielle Färbung bestimmter spezifischer Abschnitte der ophthalmischen, insbesondere der intraokulären Umgebungen.

18. Zusammensetzung nach einem der Ansprüche 1 bis 15 für ihre Verwendung bei einem Verfahren zur Diagnose durch differenzielle Färbung der pathologischen Membrane der Retina.

## Claims

1. - Stable staining composition which is suitable for sterilisation in an autoclave and which comprises in an aqueous solution:
- at least one dye,
- at least one water-soluble polyol of formula H(HCOH)ₙH, where n is a whole number greater than 3,
for use in a method selected from:
- an ophthalmic surgical treatment method applied to the human or animal body, in which said composition is applied in contact with an ophthalmic medium so as to perform in physiologically acceptable conditions a selective staining during the performance of at least one ophthalmic surgical procedure,
- a diagnostic method applied to the human or animal body (in vivo ).

2. - Composition according to claim 1, **characterised in that** it comprises at least one dye selected from the group formed by trypan blue, brilliant blue G, Evans blue, patent blue V, methylene blue, bromophenol blue and aniline blue.

3. - Composition according to one of claims 1 or 2, **characterised in that** it comprises a proportion of each dye that is acceptable for an application in contact with an ophthalmic medium.

4. - Composition according to one of claims 1 to 3, **characterised in that** it comprises a proportion of each dye in weight per unit volume of between 0.001% and 2.5%.

5. - Composition according to one of claims 1 to 4, **characterised in that** it comprises a proportion of polyol(s) that is acceptable for an application in contact with an ophthalmic medium.

6. - Composition according to one of claims 1 to 5, **characterised in that** it comprises a proportion of polyol(s) in weight per unit volume of between 0.1% and 5%.

7. - Composition according to one of claims 1 to 6, **characterised in that** n is less than or equal to 9.

8. - Composition according to one of claims 1 to 7, **characterised in that** each polyol is selected from the group formed by erythritol (n=4), xylitol (n=5), arabitol (n=5), ribitol (n=5), sorbitol (n=6), dulcitol (n=6), mannitol (n=6), volemitol (n=7), maltitol (n=9), isomaltitol (n=9) and lactitol (n=9).

9. - Composition according to one of claims 1 to 8, **characterised in that** it comprises mannitol as the polyol.

10. - Composition according to one of claims 1 to 9, **characterised in that** it additionally comprises an amount of sodium chloride such that the composition has an osmotic concentration of between 260 and 360 mOsmol/kgH₂O.

11. - Composition according to claim 10, **characterised in that** it comprises a proportion of sodium chloride in weight per unit volume of between 0.4% and 0.9%.

12. - Composition according to one of claims 1 to 11, **characterised in that** it additionally comprises a suitable buffer for maintaining a pH of between 7 and 7.5.

13. - Composition according to claim 12, **characterised in that** the buffer is H₂PO₄/HPO₄.

14. - Composition according to one of claims 1 to 13, **characterised in that** it consists of 0.001% to 2.5% (wt./vol.) of at least one dye, 0.1% to 5% (wt./vol.) of at least one water-soluble polyol, a proportion of sodium chloride such that the osmotic concentration of the composition can be adjusted to a value of between 280 mOsmol/kgH₂O and 350 mOsmol/kgH₂O, a suitable buffer for maintaining the pH of the composition at between 7 and 7.5, heavy water in a proportion of 1% to 20% (vol./vol.) and water q.s.

15. - Composition according to one of claims 1 to 14, **characterised in that** it is sterile.

16. - Composition according to one of claims 1 to 15 for use in a surgical method for treating the human or animal body, selected from:
- an ophthalmic surgical treatment method for cataract removal in which the composition is applied in contact with the outer surface of the anterior capsule of the eye,
- an ophthalmic surgical treatment method for treating pathological retinal membranes in which the composition is applied in contact with the retina,
- an ophthalmic surgical method for treating glaucoma.

17. - Composition according to one of claims 1 to 15 for use in an ophthalmic diagnostic method applied to the human or animal body by differential staining of certain specific portions of ophthalmic - in particular intraocular - media.

18. - Composition according to one of claims 1 to 15 for use in a diagnostic method, by differential staining, for pathological retinal membranes.
